# EUROPEAN PATENT SPECIFICATION

(11) **EP 3 487 562 B1**
(45) Date of publication and mention of the grant of the patent: **27.05.2020**
(21) Application number: 17752299.2
(22) Date of filing: 19.07.2017
(51) Int. Cl.: A61M 5/38, A61M 5/31

(54) **SYRINGE PLUNGER AND SYRINGE**
SPRITZENKOLBEN UND SPRITZE
PISTON POUR SERINGUE ET SERINGUE

(30) Priority: 19.07.2016 LU 93154
(43) Date of publication of application: 29.05.2019
(73) Proprietor: Abu Al-Rubb, Khalil Mahmoud, Campbell Court, Flat 11 London SW7 4PB (GB)
(72) Inventor: Abu Al-Rubb, Khalil Mahmoud, Campbell Court, Flat 11 London SW7 4PB (GB)
(74) Representative: Marks & Clerk (Luxembourg) LLP
(86) International application number: PCT/EP2017/068232
(87) International publication number: WO 2018/015438

(56) References cited:
- EP-A1- 1 529 489
- WO-A1-98/25659
- WO-A2-2004/039439
- DE-A1-102004 055 870
- US-A- 4 821 738
- US-A- 5 377 689

## Description

### TECHNICAL FIELD

Embodiments of the present invention relate to a plunger for a syringe and to a syringe.

### BACKGROUND

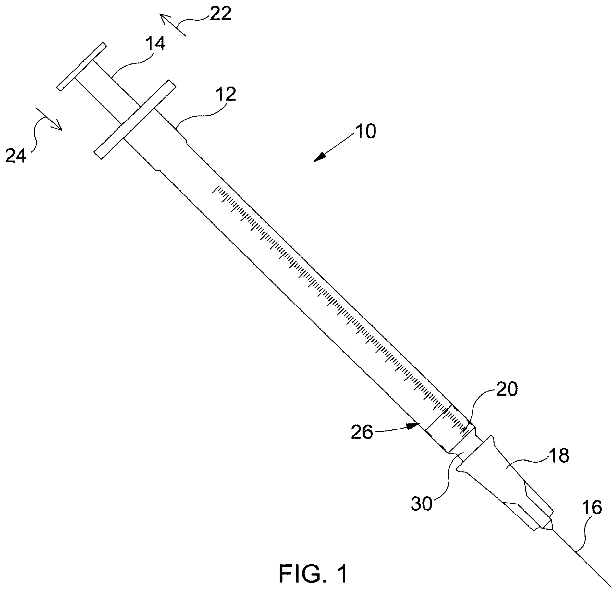

Figure 1 illustrates a syringe 10 as known in the prior art. The syringe 10 comprises a chamber in the form of a cylinder 12 and a plunger 14 arranged so that the plunger 14 reciprocates within the cylinder 12. A needle 16 is attached to the cylinder 12 by means of an applicator 18. The plunger 14 has a head 26. A seal 20 is provided on, e.g., is attached to, the head 26 of the plunger 14.

During use, the needle 16 is inserted into the medication to be administered which is dissolved in, or in suspension in, a liquid. The plunger is then drawn backwards (in the direction of arrow 22), thereby drawing the seal 20 backwards creating an under-pressure between the seal and the liquid. This, in turn, draws liquid into the cylinder 12.

During administration, the needle 16 is inserted into a vein or subcutaneously, the plunger 14 is depressed (pushed forwards in the direction of arrow 24), thereby forcing the seal 20 downwards which expels the liquid out of the needle 16 and into the patient.

As designated by reference numeral 30, a space exists between the seal 20 and the distal end of the syringe 10, e.g., tip of the needle 16. Generally, when the syringe 10 is filled, it is inverted so that the needle points upwards and the liquid is then drawn into the cylinder 12. The space 30 is filled with air prior to administration and this air, when the needle is inverted for administration, will be situated adjacent the tip of the needle. Therefore, unless any action is taken to avoid this, the aim would be injected first, prior to the liquid.

For these reasons, action of purging a syringe a well-known whereby the plunger 14 is depressed until the air has been expelled.

However, a user does not know that all of the air has been expelled until they observe liquid being expelled from the tip of the needle. Depending on the skill and dexterity of the user, this can lead to a lesser or greater expulsion of liquid. However, in each case, some of the liquid containing the medication will be expelled.

Besides that, re-usable needles like Insulin Pens keep on compiling air bubbles that are very hard to purge or get rid of totally.

This results in a significant waste of medication. Not only does this result in wasted costs, but there is an associated uncertainty regarding the dosage provided.

It is desirable to avoid or minimize the necessity to purge syringes of unwanted air, and/or provide means for improving the removal of air from the space containing the liquid to be administered.

PCT Application publication No WO 2004/039439 discloses a syringe assembly for discharging gaseous materials from a syringe. German Patent Application Publication No DE 10 2004 055 870 discloses a syringe device with a venting device. However, both these documents suffer from sub-optimal discharge of gas from the space of the syringe containing the liquid to be administered.

### SUMMARY OF INVENTION

The invention is defined in claim 1. It provides a plunger for a syringe, the plunger comprising a head for reciprocating within a chamber of the syringe, the head comprising a seal which is impermeable to liquid, but permeable to gas.

In use, reciprocation of the plunger with the chamber, e.g., depression of the plunger into the chamber, allows the syringe to deliver a shot, e.g. a volume, of liquid contained within the chamber.

Provision of a seal which is impermeable to liquid, but permeable to gas, may permit any gas, e.g., air, to pass through the head, but may prevent liquid from doing so.

The seal comprises a semi-permeable membrane. The semi-permeable membrane may typically be permeable to gas, e.g. air, but not permeable to liquid(s), e.g. to an aqueous medium such as an aqueous solution or dispersion/emulsion.

Typically, the membrane may be made from a synthetic material, e.g. from a polymeric material. The membrane may be made from polyethylene, polypropylene, or the like. The membrane may be made from a nonwoven polypropylene material. For example, the membrane may be made from a material sold by Proctor Group Ltd (UK) under the trade name Roofshield. It will be appreciated that other types of semi-permeable membranes may be used, which may depend for example on the dimensions of the syringe, on the intended use for the syringe, on the liquid to be administered, and/or on the chemical and physical properties on the membrane.

Advantageously, the membrane may be located at or near an end portion of the head and/or seal. The membrane may be located at or near a portion of the head and/or seal nearest a needle end thereof. The plunger, head and/or seal is configured such that the membrane forms or defines substantially the entire surface of the plunger and/or of the seal in contact with and/or exposed to the space, e.g. liquid and/or gas contained therein. The portion of the plunger, head and/or seal in contact with and/or exposed to the space may be defined by, e.g. may be exclusively defined by, the membrane, e.g. a surface of the membrane.

During use of the syringe, when the syringe is inverted after having filled, any gas, e.g. air, may be located adjacent to the seal, e.g. membrane thereof. When a user depresses the plunger, the action may cause the seal, e.g. membrane, to press against the gas, e.g. air, located in the space. This force causes the gas to pass through the membrane of the seal. In this manner, some or all of the gas may be removed without having to purge the syringe before administration, thereby avoiding waste of any of the medication involved and/or saving time. Further, when the membrane defines the entire surface of the plunger, head and/or seal contact with and/or exposed to the space, no further part of the plunger may interfere with evacuation of the gas through the seal, thus optimising the gas removal performance of the present arrangement.

In an embodiment, any gas having passed through the seal and/or membrane may be evacuated from the syringe.

In an embodiment, any gas having passed through the seal and/or membrane may be contained in a cavity defined by the syringe and/or plunger.

In an embodiment, the syringe, e.g. plunger, may include a reservoir located near its head, e.g. adjacent to the seal, e.g. on a side thereof opposite the membrane. This may provide a means for storing any gas having passed through the seal and/or membrane.

In an embodiment, a portion of the head and/or seal may be configured to receive, accommodate, and/or store gas which may be located in the space between the seal and the liquid in the chamber, and which may pass through the membrane. The head and/or seal, e.g. a portion of the seal in contact with the membrane, may be made from a porous material. The head and/or seal, e.g. a portion of the seal in contact with the membrane, may be made from a foam material capable of receiving, accommodating, and/or storing gas that passes through the membrane. By such provision, the syringe may advantageously provide effective purging and storing of any gas contained in the space. The head and/or seal, e.g. a portion of the seal in contact with the membrane, may be made from foam plastic and/or foam rubber.

The membrane may be attached to a support member of the plunger, e.g. head thereof. In an embodiment, the membrane may be permanently attached to the support member, e.g. may be bonded, welded, or the like, to the support member. Advantageously, the membrane may be ultrasonically welded to the support member. By such provision, effective bonding of the membrane to the support member may be achieved, without compromising the effectiveness of the membrane or requiring the use of adhesives.

The support member may be provided by an end portion of the plunger, e.g. head. In such instance the seal may be defined by the membrane. The support member may be provided by an end portion of the plunger, e.g. seal. In such instance the seal may further comprise the membrane and be act as a/the support member for the membrane.

A further embodiment of the invention defined in claim 9 extends to a syringe comprising a chamber for receiving a liquid to be dispensed and a piston for reciprocating with the chamber so that liquid contained within the chamber is expelled by movement of the piston, the piston comprising a plunger as herein described.

The membrane may be dimensioned in accordance with a size of the chamber to accommodate gas which may be stored in the chamber together with the liquid.

### BRIEF DESCRIPTION OF DRAWINGS

Embodiments of the invention are described with reference to the accompanying schematic diagrams in which:
Figure 1 is an illustration of a syringe as known in the art;
Figures 2 and 3 are illustrations of syringes according to embodiments of the invention.

### DETAILED DESCRIPTION OF EMBODIMENTS

Embodiments of the invention are described hereafter with reference to the accompanying diagrams.

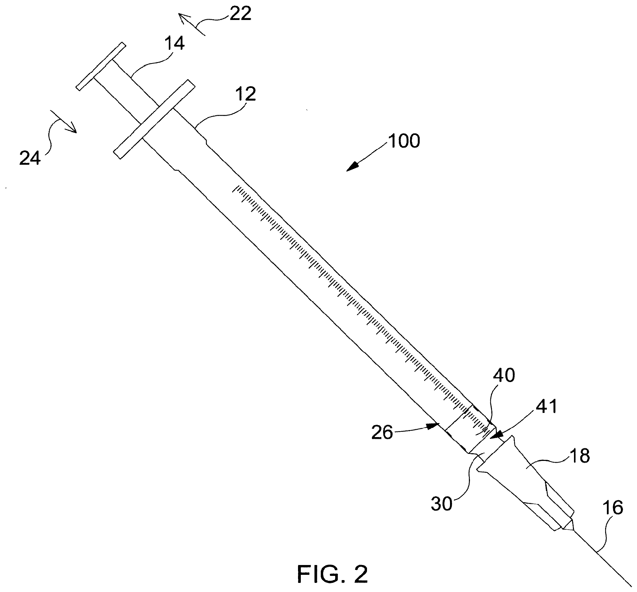

Figure 2 illustrates a syringe 100 according to a first embodiment. In this embodiment of the invention, the seal 20 is replaced by a seal 40 at least a portion of which is permeable to gas, but not to liquid. In one embodiment, the seal 40 is provided by a semi-permeable membrane 41 which is permeable to gas but not to liquid.

Typically, the membrane 41 is made from a synthetic material, e.g. from a polymeric material. The membrane 41 may be made from polyethylene, polypropylene, or the like. The membrane 41 may be made from a nonwoven polypropylene material. In this embodiment, the membrane is a material sold by Proctor Group Ltd (UK) under the trade name Roofshield.

Advantageously, the membrane 41 is located at or near an end portion of the seal. The membrane is located on a portion/surface of the seal 40 nearest a needle end thereof. The plunger 14 and/or seal 40 is configured such that the membrane 41 forms or defines the entire surface of the plunger in contact with the space 30, e.g. with a liquid and/or gas contained therein.

During use of the syringe 100, when the syringe is inverted after having filled, the air will be located adjacent to the seal 40. When a user depresses the plunger 14, the action causes the seal 40 to press against the air located in space 30. This force creates the force necessary for the gas to pass through the membrane 41 of the seal 40. In this manner, all the gas may be removed without having to purge the syringe and thereby avoiding the waste of any of the medication involved.

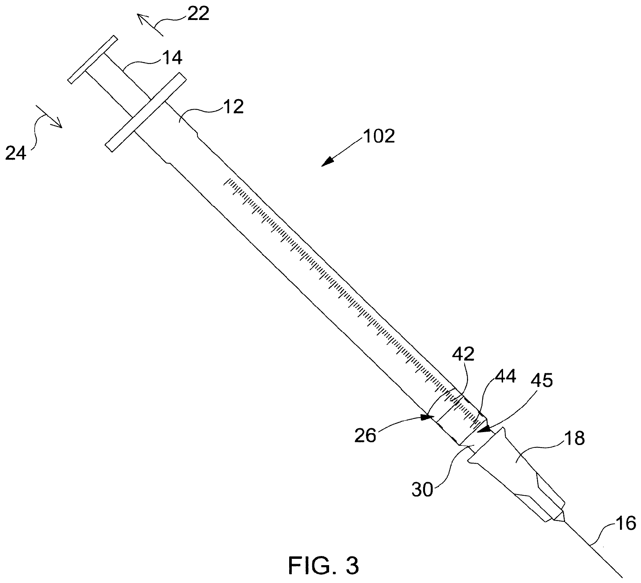

Figure 3 illustrates a further embodiment of the invention. In this embodiment, syringe 102 includes a reservoir 42 located adjacent to the seal 44 on a side of the seal opposite the space 30 and membrane 45.

The seal 40 of the embodiment of Figure 2 is dimensioned to accommodate the air which may be located between the seal 40 and the liquid in the chamber 12. The plunger 40, e.g. seal thereof may for example be made of hard foamed rubber to host the air that passes through the membrane 45 and into its voids

The embodiment of Figure 3 differs from that of Figure 2 in that any excess air passes through the seal 44 and is then contained within reservoir 42. The seal 44 also comprises a material such as a membrane 45 which is permeable to gas, but not to liquid.

It will be appreciated that the dimensions of the seal 40 as illustrated in Figure 2 and/or seal 44 as illustrated in Figure 3 may be chosen in dependence on the diameter and length of the piston 14 and chamber 12 to accommodate the typical air volumes which may be involved in normal administration of a medication.

## Claims

1. A plunger for a syringe, the plunger comprising a head for reciprocating within a chamber of the syringe, the head further comprising a seal which is impermeable to liquid, but permeable to gas, the seal comprising a semi-permeable membrane, **characterized in that** the membrane forms or defines substantially the entire surface of the plunger and/or of the seal in contact with and/or exposed to a space of the chamber between the seal and an end of the chamber.

2. The plunger according to claim 1, wherein the membrane forms or defines substantially the entire surface of the plunger and/or of the seal in contact with liquid and/or gas contained in the chamber.

3. The plunger according to any of claims 1 or 2, wherein the portion of the plunger, head and/or seal in contact with and/or exposed to the space and/or to liquid and/or gas contained in the chamber, is defined by the membrane.

4. The plunger according to any one of claims 1 to 3, wherein the head comprises a reservoir in fluid communication with the seal to accommodate gas passing through the seal.

5. The plunger according to claim 4, wherein the reservoir is located on a side of the seal opposite the chamber and/or opposite the membrane.

6. The plunger according to any one of claims 1 to 5, wherein the head and/or seal is configured to receive, accommodate, and/or store gas which passes through the membrane.

7. The plunger according to claim 6, wherein at least a portion of the head and/or seal in contact with the membrane is made from a porous material.

8. The plunger according to claim 7, wherein at least a portion of the head and/or seal in contact with the membrane is made from foam rubber and/or foam plastic.

9. A syringe comprising a chamber for receiving a liquid to be dispensed and a piston for reciprocating with the chamber, the piston comprising a plunger according to any of claims 1 to 8.

## Patentansprüche

1. Druckkolben für eine Spritze, wobei der Druckkolben einen Kopf zum Hinundhergehen innerhalb einer Kammer der Spritze umfasst, wobei der Kopf ferner eine Dichtung umfasst, die undurchlässig für Flüssigkeit, aber durchlässig für Gas ist, wobei die Dichtung eine halbdurchlässige Membran umfasst, **dadurch gekennzeichnet, dass**
die Membran im Wesentlichen die gesamte Oberfläche des Druckkolbens und/oder der Dichtung in Berührung mit und/oder freigelegt zu einem Raum der Kammer zwischen der Dichtung und einem Ende der Kammer bildet oder definiert.

2. Druckkolben nach Anspruch 1, wobei die Membran im Wesentlichen die gesamte Oberfläche des Druckkolbens und/oder der Dichtung in Berührung mit Flüssigkeit und/oder Gas, die in der Kammer enthalten sind, bildet oder definiert.

3. Druckkolben nach einem der Ansprüche 1 oder 2, wobei der Abschnitt des Druckkolbens und/oder der Dichtung in Berührung mit und/oder freigelegt zu dem Raum und/oder Flüssigkeit und/oder Gas, die in der Kammer enthalten sind, durch die Membran definiert wird.

4. Druckkolben nach einem der Ansprüche 1 bis 3, wobei der Kopf ein Reservoir in Fluidverbindung mit der Dichtung umfasst, um Gas aufzunehmen, das durch die Dichtung hindurchgeht.

5. Druckkolben nach Anspruch 4, wobei das Reservoir auf einer Seite der Dichtung, gegenüber der Kammer und/oder gegenüber der Membran, angeordnet ist.

6. Druckkolben nach einem der Ansprüche 1 bis 5, wobei der Kopf und/oder die Dichtung dafür konfiguriert sind, Gas zu empfangen, aufzunehmen und/oder zu speichern, das durch die Membran hindurchgeht.

7. Druckkolben nach Anspruch 6, wobei mindestens ein Abschnitt des Kopfes und/oder der Dichtung in Berührung mit der Membran aus einem porösen Werkstoff hergestellt ist.

8. Druckkolben nach Anspruch 7, wobei mindestens ein Abschnitt des Kopfes und/oder der Dichtung in Berührung mit der Membran aus einem Schaumgummi und/oder Schaumkunststoff hergestellt ist.

9. Spritze, die eine Kammer zum Aufnehmen einer Flüssigkeit, die abgegeben werden soll, und einen Kolben zum Hinundhergehen innerhalb der Kammer umfasst, wobei der Kolben einen Druckkolben nach einem der Ansprüche 1 bis 8 umfasst.

## Revendications

1. Plongeur pour une seringue, le plongeur comprenant une tête destinée à effectuer un mouvement de va-et-vient dans une chambre de la seringue, la tête comprenant en outre un joint d'étanchéité imperméable au liquide, mais perméable au gaz, le joint d'étanchéité comprenant une membrane semi-perméable, **caractérisé en ce que** :
la membrane forme ou définit sensiblement l'ensemble de la surface du piston, et/ou du joint d'étanchéité en contact avec et/ou exposé à un espace de la chambre entre le joint d'étanchéité et une extrémité de la chambre.

2. Plongeur selon la revendication 1, dans lequel la membrane forme ou définit sensiblement l'ensemble de la surface du plongeur et/ou du joint d'étanchéité en contact avec le liquide et/ou le gaz contenu dans la chambre.

3. Plongeur selon l'une quelconque des revendications 1 ou 2, dans lequel la partie du plongeur, de la tête et/ou du joint d'étanchéité en contact avec/ou exposée à l'espace et/ou au liquide et/ou au gaz contenu dans la chambre est définie par la membrane.

4. Plongeur selon l'une quelconque des revendications 1 à 3, dans lequel la tête comprend un réservoir en communication de fluide avec le joint d'étanchéité pour recevoir le gaz passant à travers le joint d'étanchéité.

5. Plongeur selon la revendication 4, dans lequel le réservoir est agencé sur un côté du joint d'étanchéité opposé à la chambre et/ou opposé à la membrane.

6. Plongeur selon l'une quelconque des revendications 1 à 5, dans lequel la tête et/ou le joint d'étanchéité est configuré pour recevoir, loger et/ou stocker le gaz passant à travers la membrane.

7. Plongeur selon la revendication 6, dans lequel au moins une partie de la tête et/ou du joint d'étanchéité en contact avec la membrane est fabriquée à partir d'un matériau poreux.

8. Plongeur selon la revendication 7, dans lequel au moins une partie de la tête et/ou du joint d'étanchéité en contact avec la membrane est fabriquée à partir de caoutchouc mousse et/ou de plastique mousse.

9. Seringue comprenant une chambre pour recevoir un liquide devant être administré et un piston destiné à effectuer un mouvement de va-et-vient dans la chambre, le piston comprenant un plongeur selon l'une quelconque des revendications 1 à 8.
